Europäisches Patentamt

**European Patent Office**

Office européen des brevets

(11) Publication number : **0 399 321 B1**

(12) **EUROPEAN PATENT SPECIFICATION**

(45) Date of publication of patent specification :
23.06.93 Bulletin 93/25

(51) Int. Cl.⁵ : **A61K 35/16**

(21) Application number : **90108993.8**

(22) Date of filing : **12.05.90**

(54) **Gel filtration of heat treated factor VIII.**

(30) Priority : **24.05.89 US 356315**

(43) Date of publication of application :
**28.11.90 Bulletin 90/48**

(45) Publication of the grant of the patent :
**23.06.93 Bulletin 93/25**

(84) Designated Contracting States :
**AT BE CH DE DK ES FR GB GR IT LI LU NL SE**

(56) References cited :
**EP-A- 0 047 216**
**WO-A-84/00757**
**WO-A-84/03628**
**US-A- 3 631 018**
**US-A- 4 069 216**
**US-A- 4 170 639**
**US-A- 4 543 210**
**PATENT ABSTRACTS OF JAPAN vol. 7, no.**
**167 (C-177)(1312) 22 July 1983**
(73) Proprietor : **MILES INC.**
**Fourth and Parker Streets P.O. Box 1986**
**Berkeley California 94701 (US)**

(72) Inventor : **Goelker, Christian, Dr.**
**Am Rohm 45**
**W-5600 Wuppertal 1 (DE)**
Inventor : **Bockskopf, Bernd, Dr.**
**Rabensweg 46**
**W-5600 Wuppertal 1 (DE)**
Inventor : **Brockway, William**
**3732 Victor Avenue**
**Oakland, California 94619 (US)**
Inventor : **Seng, Richard L.**
**P.O. Box 2038**
**Guerneville, California 95446 (US)**

(74) Representative : **Dänner, Klaus, Dr. et al**
**c/o Bayer AG Konzernverwaltung RP**
**Patentabteilung**
**W-5090 Leverkusen 1 Bayerwerk (DE)**

## Description

The present invention relates to methods of preparing antihemophilic factor (AHF) from human plasma. AHF is now known to consist of several components, the component which is active in treating hemophilia A being Factor VIII:C.

Numerous patents and publications exist which relate to the preparation of AHF concentrates as part of the fractionation of human plasma. Such processes have been in commercial use for approximately 20 years, and numerous processing variations have been described, the vast majority of which are directed to the inherent problems in such processes, namely virus safety, yield, and specific activity of the resultant concentrate. Specific activity refers to the coagulation activity of the Factor VIII, expressed in international units, according to a currently accepted standard, per mg of total protein.

Although gel filtration of chromatography, except for affinity chromatography such as described in Zimmerman et al, Re. 32,011 (U.S. 4,361,509) is not, to the inventors' knowledge, in current commercial use, several chromatography processes have been described. It is important to note that all affinity chromatography or rDNA processes will result in AHF having detectible amounts of non-human protein.

For example, PCT Application Publication No. WO 86,04486 discloses a method for purifying AHF by "hydration additives", i.e. using column chromatography in the presence of sugars, polyols, amino acids or salts. The low yield of prior art chromatography processes is described. The hydration additives serve to stabilize the AHF. Cryoprecipitate is dissolved in a buffer, aluminum hydroxide may be added and the supernatant collected. A PEG precipitation step is carried out. One or two column chromatography steps are then carried out, using resins such as QAE Sephadex® A-25, QAE-Sepharose® 4B or aminohexyl (A-H) Sepharose. The first chromatography step is based on anion exchange, the second on hydrophobic affinity.

Andersson, EP 197901, discloses a method for preparing fragments of AHF using immunoaffinity chromatography followed by HPLC on an anion-exchange adsorbent. The anion exchange adsorbent may be Mono Q gel or TSK DEAE 5 PW gel. Fragments are then obtained by incubation with thrombin.

Johnson, U.S. Patent 4,397,841, discloses preparation of Factor VIII:C by fractionation of plasma with a sequence of adsorption steps employing polyelectrolyte copolymers in the presence of heparin. A suitable resin is a copolymer of ethylene and maleic anhydride.

Chavin et al., U.S. Patent 4,495,175, disclose the preparation of highly purified AHF from AHF concentrate. The concentrate is subjected to a separation on the basis of Stokes' radius, which may be accomplished, for example, by gel permeation chromatography on cross-linked agarose (such as Biogel® A-15M or Sepharose® EL-4B). The pool is then concentrated by precipitation or diafiltration; calcium or magnesium cations are added to reduce the Stokes' radius, and a separation on the basis of Stokes' radius is again carried out.

Various other steps such as are employed in the present process have been disclosed in the prior art. However, as described below, novel and unexpected results and modifications are embraced by the present invention.

Liu et al., U.S. Patent 4,170,639, for example, disclose a process for preparing AHF comprising the steps of subjecting resolubilized cryoprecipitate to aluminum hydroxide adsorption at an acid pH and 4° C; filtration; and, optionally, ultrafiltration.

Rasmussen et al., U.S. Patent 4,650,858, disclose a process for producing AHF using a 4% PEG precipitation step at 18 - 22° C to remove fibrinogen. This is followed by a second PEG precipitation step at 18 - 22° C with 12% PEG in the presence of an amino acid such as 2M glycine to precipitate the AHF.

Shanbrom, U.S. Patent 4,069,216, discusses PEG precipitation as disclosed in the prior art, e.g. his 3,631,018, wherein room temperature precipitation necessitates a subsequent washing and/or glycine or alcohol precipitation step, since the PEG is used in high concentrations (10 - 12%). Cold precipitation using lower concentrations of PEG (2½%) resulted in a less purified product.

Liautaud et al., U.S. Patent 4,387,092, disclose an improvement to Shanbrom 4,069,216 in that the fibrinogen precipitation step is carried out at below 15° C with less than 4% polyol.

Polson, U.S. Patent 3,415,804, discloses plasma fractionation with PEG at room temperature, around 20° C. At 0 - 4% PEG, fibrinogen precipitated, gamma globulin precipitated at 4 - 8%, beta globulin at 8 - 12% and alpha-1 and alpha-2 globulins and albumins at greater than 12% PEG.

Finally, relevant prior art exists with regard to virus inactivation of AHF concentrates.

Neurath et al., U.S. Patent 4,540,573, disclose viral inactivation of Factor VIII preparations through the use of tri-(n-butyl) phosphate (TNBP). It is there suggested that TNBP may be added to the plasma pool, and AHF can be separated from TNBP by a precipitation step, such as with glycine. In the Examples, TNBP is added to AHF solutions having 8 - 10 u/mL F.VIII activity.

Andersson et al., U.S. Patent 4,168,300, disclose a method of removing hepatitis virus from plasma by adsorbing the HBsAg, or Au-antigen onto a beaded agarose gel, or a copolymer gel, having a hydrophobic lig-

and coupled thereto.

Lembach, U.S. Patent 4,534,972, discloses the use of copper phenanthroline for viral inactivation of AHF preparations. The substance is added after fractionation and may be removed by diafiltration.

High yields of antihemophilic factor (AHF) can be achieved using milder processing steps in combination with a heat treatment for viral inactivation and gel filtration to provide highly purified AHF which is substantially free from infectious agents, without substantial loss of therapeutic or immunological activity.

In one particular aspect of the present invention, cryoprecipitate is recovered by centrifugation from thawed pools of fresh frozen human plasma. Extraneous non-AHF proteins are removed by acid precipitation and adsorption with $Al(OH)_3$ and PEG precipitation under conditions which produce high precipitation of non-AHF proteins. As a result, a chill step is not needed. The AHF is then precipitated with glycine and sodium chloride. Solubilized AHF concentrate is then treated to remove additional impurities, heat treated for viral inactivation and then gel-filtered. The preferable gel has a 5 million dalton cut-off and 200 - 400 mesh.

AHF is then lyophilized after sterile filtration in the presence of albumin.

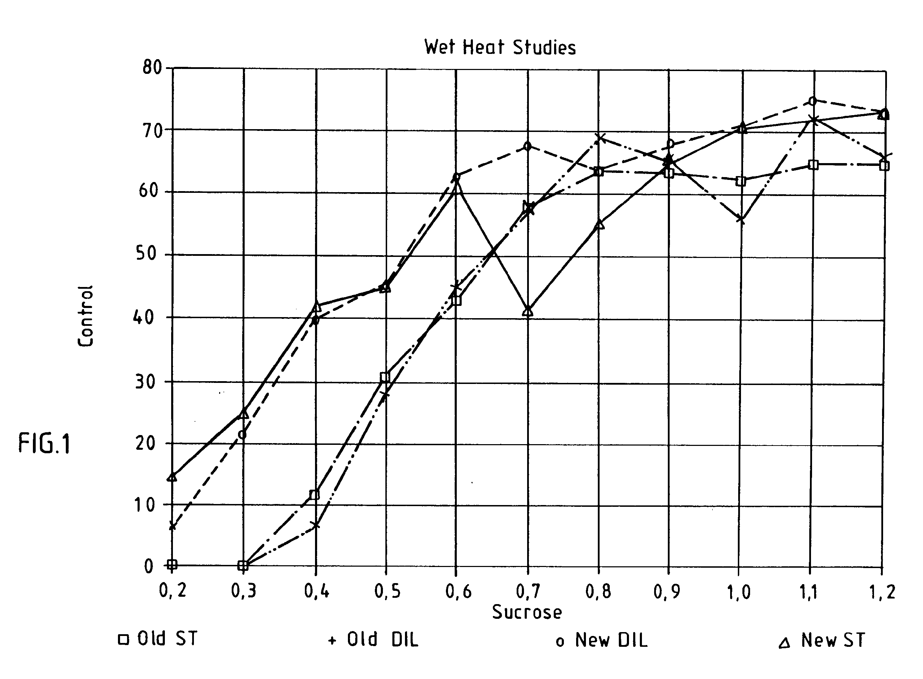

Figure 1 depicts graphically the effect of heat on F.VIII activity in the presence of various amounts of sucrose in the preparations of the present invention.

Example 1

Cryoprecipitate (cryo) from a normal plasma pool of plasmapherised donors was dissolved by adding 3 Kg of WFI/Kg cryo. The WFI can include up to 60 u/ml of sodium heparin before the cryo is added. 30.2 Kg of cryo was added to 90.5 Kg WFI at a temperature of 27°C and mixed to dissolve the cryo. The temperature range of WFI is preferably 17 - 37° C, most preferably 24 - 30° C. Although the ratio of 1 part cryo/3 parts WFI are used in the example, 1 part cryo/4 parts WFI can be used to obtain the same results.

The cryo/WFI mixture was stirred for 30 minutes until dissolved. The resulting temperature was 21° C, a preferable range being 18 - 25° C. The $A_{280}$ was 41.2, a preferable range being 38 to 44 and a pH of 7.75, preferable range being 7.6 - 8.0.

The pH of the dissolved cryo/WFI solution was adjusted to 7.0, the preferable range being 6.0 - 8.0, most preferably 6.8 - 7.2 with the dropwise addition of 270 ml of 1N acetic acid and the suspension was stirred for 15 minutes. The average yield was 116% with a yield range of 110 - 127%. The apparent yield increase is due to removal of fibrinogen and other components which interfere in the AHF assay. The foregoing steps may be carried out at room temperature to avoid a chill step and additional precipitation, and to avoid protein denaturation.

For the adsorption step, 4826 ml of aluminum hydroxide, $Al(OH)_3$, gel was added to the acid cryo suspension and stirred for 10 minutes to bind the vitamin K dependent factors. The amount of $Al(OH)_3$ gel represents 160 ml of $Al(OH)_3$ gel per Kg of starting cryo, a preferable range being 100 - 250 ml of $Al(OH)_3$ gel per Kg of cryo. The average yield across this step is 94% with a yield range of 90 - 100%.

For polyethylene glycol (PEG) precipitation, 3.6 Kg of PEG 3350 (3% PEG) was added to the $Al(OH)_3$ - acid cryo suspension and the pH was readjusted to 7.06 with 16 ml of 1 M acetic acid. The pH range being 6.0 - 8.0, more preferably 6.8 - 7.3. The concentration of PEG can range from 2.5 - 5%. The suspension was stirred for 23 minutes before centrifugation. The temperature of the suspension was 21.5° C, preferably not less than 10° C.

The suspension was centrifuged using a Westphalia BKA-6 centrifuge at 4 l/min flow rate, the preferable range being 2 - 6 l/min. The effluent temperature was maintained at 20 ° C, the preferable range being 18 - 25° C with the influent temperature of 21.5° C, the preferable range being 20 - 25° C.

The resulting precipitate was harvested, weighed and discarded. The 10.7 Kg precipitate represented 35.4% of the starting cryo. The average precipitate being 32.4% with a range of 29.0 - 36.3%.

The PEG effluent weighed 116.6 Kg, had an $A_{280}$ of 10.4, pH 7.26 at a temperature of 20° C. The temperature range is preferably 20 - 23° C, if necessary a warming step can be added for a PEG effluent having a temperature lower than 20° C. The average yield of AHF recovered through the PEG step was 78% with a range of 74.3 - 86.1.

An important advantage is recognized in the elimination of the chill step conventionally used in the PEG precipitation. This is an advantage because a chill step will precipitate fibrinogen, fibronectin, etc., but also will precipitate AHF, reducing yield.

The resulting final AHF paste obtained is a very good working paste weight to avoid loss of AHF or high volume of column gel. Too low a paste weight results in loss of AHF, too high a paste weight requires a large volume of column gel for the gel filtration step.

To the PEG effluent was added 15.2 Kg of solid L-glycine (or 13% glycine) while maintaining the pH at 7.0, preferable range 6.0 - 8.0, by the addition of 200 ml of 1 M sodium hydroxide. The addition of glycine low-

3

ered the temperature of the PEG effluent to about 15° C. The solution was warmed to 20° C, the preferable range being 20 - 23° C. The solution was stirred for 20 minutes until dissolved.

To the glycine-PEG effluent solution was added 16.3 Kg solid NaCl (or 14% NaCl) while maintaining the pH at 7.0, the preferable range being 6.0 - 8.0, with 200 ml of 1 M NaOH. The final temperature was adjusted to 20° C the preferable being 20 - 23° C. The final pH was 7.03 with a range of 6.9 - 7.2. The solution was stirred for 25 minutes until dissolved.

The glycine-NaCl - PEG effluent was centrifuged to remove the AHF paste at the flow rate of 2.0 l/min. The inlet temperature was 20° C, the preferable range being 20 - 23° C The effluent temperature was maintained at 21 - 22° C, the preferable range being 18 - 25° C. The $A_{280}$ of the effluent was measured at 9.1 and the effluent discarded.

The harvested AHF paste weighed 1.03 Kg. It was dissolved in a buffer containing 0.02 M L-histidine, 0.10 M ammonium formate, 1.5% mannitol, 0.001 M $CaCl_2$ at a pH of 7.0, the preferable range being 6.9 - 7.1. The buffer can contain not more than 0.2 M ammonium formate, 0.06 M L-histidine, 0.003 M $CaCl_2$ and 3% mannitol. The buffer should minimize the protein modification, i.e., non-specific binding of copper phenanthroline. Alternative buffers can be used, for example: Water for Injection (WFI); O.15 M NaCl, 0.00 1 M, $CaCl_2$, pH 7.2; 0.05 M imidazole, pH 7.0; or 0.05 M Tris HCl/0.15 M NaCl, pH 7.0, or 0.02 M L-histidine, 0.15 M NaCl, 0.001 M $CaCL_2$, pH 7.2.

The resulting dissolved AHF concentrate had an $A_{280}$ of 33.2, a weight of 3.84 Kg and a potency of 432 u/ml. In previous runs the average potency was 232 u/ml, the range was 130 - 287.5 u/ml. Because of this much higher than normal potency as compared to previous PEG precipitation methods, the chemical treatment for viral inactivation and gel filtration steps are performed without the necessity of a further concentration step, as previously required, such as ultrafiltration. The recovery of units of AHF as compared to the dissolved cryo was 63.2%, the average 67.3% with the range being 56.7 - 71.8° C. In previous runs, the yield of AHF from the PEG effluent to the dissolved AHF concentrate was an average of 78.3% with the recovery range being 68.3 - 90.0%.

The solubilized AHF can be frozen at -20° C or colder and stored at -70° C or processed immediately.

The frozen (-70°) AHF concentrate was thawed in a 27° C water bath for approximately 4 hours until the temperature of the thawed AHF concentrate was 25.2° C.

It is important to note that all steps up to the optional freeze step were carried out at room temperature.

A forty-fold concentrated copper phenanthroline (CuPH) buffer was prepared by mixing 10 ml 0.1 M histidine, 8 ml of 0.01 M copper sulfate pentahydrate and 8 ml of 0.5 M 1,10 phenanthroline. The final volume was adjusted to 200 ml. with WFI. A volume of 87.5 ml of the CuPH buffer was added to 3500 ml of the AHF concentrate in an autoclaved, enclosed reactor. The enclosed CuPH reactor was constructed to rotate end to end to wet all internal surfaces. Oxygenation was delivered by diffusion through 25 feet of silastic medical grade tubing wound around a holder inside the reactor. During the reaction, medical grade oxygen at 2.5 psi was delivered to the reactor, which rotated at a rate of 3 rpm.

The CuPH reaction was started by the addition of 35 ml of 0.2 M L-cysteine hydrochloride monohydrate as described in the above referenced U.S. Patent No. 4,534,972. As described in this patent, a second addition of 17.5 ml of 0.2 M L-cysteine hydrochloride was injected after the first addition was exhausted. The addition was also oxidized.

Before emptying and rinsing the reactor, the reactor was transferred to a virus free room, and the outside of the reactor disinfected with sodium hypochloride. The CuPH reaction mixture was warmed to not more than 37° C and prefiltered. The prefiltering step is not required but is utilized to preserve the lifetime of the gel filtration column. The prefiltered AHF was pumped onto 4 X 16 l Pharmacia stack column packed with Bio-Gel® A-5M (100 - 200 mesh) at 8.4 l/h, the loading range being 6 - 12 l/h.

Four Pharmacia® KS 370/15 stack sections were connected in series and run from bottom to top, using a MasterFlor pump.

The AHF recovered from the CuPH reactor was 90% of the AHF in the AHF concentrate, the average being 88.3% with a range of 80.7 - 93.5%. In open CuPH reactors, such as in stirred beakers, an average recovery of 93.7% with a range of 88 - 98.7% was attained. These are very high yields compared to more conventional wet heat viral inactivation steps where approximately 25% loss of AHF activity is evidenced through pasteurization, diafiltration and ultrafiltration. Further, the mild processing steps also minimize the likelihood of deleterious effects on proteins.

The stack column was equilibrated with a buffer containing 0.15 M NaCl, 0.001 M $CaCl_2$, pH 7.16 at 22° C. Ranges for the buffer being not more than 0.2 M NaCl, not more than 0.003 M $CaCl_2$, pH 6.8 - 7.8, and temperature 16 - 26° C. After the total of 3.9 Kg of the CuPH treated AHF had been pumped into the column, the same buffer used to equilibrate the column was used as an elution buffer. The elution buffer was pumped into the column at a flow rate of 9.0 l/h, the range being 6 - 12 l/h. Alternative buffers can be used, for example, 0.05 M Trizma base, 0.15 M NaCl, 0.001 M CaCl$_2$, pH 7.4 or 0.02 M L-histidine, 0.15 M NaCl, 0.001 M CaCl$_2$, pH 7.2. Since the elution buffer is present in the final container, it should be non-toxic and the ionic strength should not be so high that it dissociates the AHF from the von Willebrand factor.

The prefiltered CuPH treated AHF, 3.9 Kg, was gel filtered using 64 l of Bio-Rad's Biogel® A5M (100-200 mesh) column equilibrated with the above described elution buffer, with application of 6.1% of the gel volume, the preferable range being 5 - 8.0% of the gel volume for efficient separation and yield. More gel volume would result in less potency in the AHF pool, less gel volume would lower the yield. The time between applying the AHF to the column until the beginning of the collection of the AHF pool was 2.35 hours. The collection of AHF pool was begun when the UV monitor indicated that A$_{280}$ was eluting. The void volume (Vo) was 20.03 Kg.

The AHF pool was collected until direct A$_{280}$ spectro-photometic reading indicated that an A$_{280}$ of 2.0 was obtained. A weight of 14.8 Kg of AHF pool was collected. Gel filtration is an effective means of removing the copper phenanthroline reactants, as evidenced by the fact that once the AHF pool is eluted, the pink CuPH reactants are still less than one-half way through the column. Furthermore, large proteins such as fibrinogen, and fibronectin are also separated out by gel filtration.

A series of experiments were conducted to confirm that CuPH reactants were removed and to evaluate residual levels of phenanthroline (PH) using radio-labelled [14]C. $^{14}$C-PH was prepared and used to monitor the removal of the compound during various process steps. These results indicated that gel filtration is an effective procedure for removal of free PH from AHF and other proteins. Further studies showed that the association of PH with protein was decreased approximately 4 to 5 fold when the reaction was run in the presence of ammonium formate, histidine and mannitol. These compounds were added to the process to minimize the presence of small residual amounts of PH associated with the protein.

The recovered AHF pool had a pH of 6.85, and A$_{280}$ of 1.21, weight of 14.8 Kg and potency of 56.6 u/ml. This yields a specific activity of 56.6/1.21 = 46.8 units/A$_{280}$ unit and a purification of 46.8/13 (for AHF concentrate) = 3.6 fold. The yield through the column was 75.5%, with an average yield of 79.5% and a range of 70.1 - 89.9 from previous runs. Due to the high potency of the AHF pool (56.6 u/ml), no ultrafiltration was performed. In fact, the AHF pool had to be diluted with column buffer down to approximately 35 u/ml for further processing. However, if a higher final container concentration is desired, the AHF pool can be easily ultrafiltered to 100 to 300 u/ml.

Although this particular run of the AHF pool was not frozen, previous AHF pools from the gel filtration column have been frozen and stored at -70°, as a hold step until bulked and freeze dried.

Normal serum albumin was added such that the calculated final container potency would be approximately 25 u/ml. 492 ml of 25% albumin was added to aid in final container reconstitution. This amount of albumin corresponds to 5 mg albumin per ml of AHF solution, with a range of 10 mg albumin, more preferably 3 - 5 mg albumin/ml of AHF. In addition to albumin, the final container can contain stabilizing agents such as 0.2 M glycine and 0.001 M CaCl$_2$ or 0.15 M NaCl and 0.001 M CaCl$_2$.

The human serum albumin (HSA) pool was sterile filtered using a 10 x 2.54cm (10 inch) Duofine, a 12 x 2.54cm (12 inch) CWSS and as a sterile filter, a 10 x 2.54cm (10 inch) Millipore TP. The sterile filters were rinsed with fresh column buffer to a target bulk weight of 14.6 Kg. The AHF recovery through the sterile filtration was 91.5%, with an average of 85%, and a range of 78 - 92.6%. The A$_{280}$ of the sterile filtered AHF was 5.15.

The sterile AHF - HSA solution was mixed in a sterile bulk container and aseptically filled in 50cc bottles, 20 ml in each bottle, and placed in a production freeze dryer and lyophilized. The yield across freeze drying was 89.8% with an average of 89.4% and a range of 78 - 111%.

The final containers were subjected to extensive analysis for quality control, and demonstrated a stable, pyrogen-free, sterile, safe preparation with very low levels of IgG, IgM, IgA, fibrinogen and fibronectin.

The concentration of the final container was 610 AHF units/20 ml, with a specific activity of 5.7 AHF units/mg protein and very low levels of copper and phenanthroline were detected.

## Example 2

Samples from the same lot of low specific activity, ultrafiltered AHF final container concentrate were gel filtered over various gel filtration (GF) columns and compared for their efficiency in separating AHF from the remainder of the other contaminants. The various gel filtration resins were poured into 2.6 X 25 cm columns and 10 ml of the concentrate applied and gel filtered. The results are shown in Table 1. Pool 1 represents the AHF pool collected by following A$_{280}$ from rise to 2.0, as described above. The Pool 2 represents all the rest of the A$_{280}$ eluted from the particular gel filtration column. The total recovery represents the sum of the yields in Pool 1 and 2.

From the table it can be seen that Pharmacia Cl-4B®, Bio-Gel® A-15M, and LKB Ultrogel® A4 also give results that are similar to those obtained with Bio-Rad's BioGel® A5M. In separate experiments it was found that the 100-200 mesh Bio-Gel® A5M resin was optimal compared to the other two meshes. Mesh refers to U.S. Standard Wet Mesh Designation (hydrated).

These gels are selected to have fractionation ranges which enable the AHF/von Willebrand complex to be separated from the majority of other impurities, such as fibrinogen, fibronectin, etc.

Some of the gels shown in Table 1 resulted in less than 50% yield of AHF, presumably because of poor fractionation ranges. All would serve to remove chemical reactants from the described viral inactivation steps, since such reactants have an MW less than 300 d.

The Pharmacia® gels are all cross-linked beaded agarose. The Bio-Gel® resins are all agarose-based gels. LKB Ultrogel A4R® has 4% agarose beads. The Fractogels are hydophilic semi-rigid spherical gels prepared from vinyl polymers. The CPG series refers to controlled pore glass beads.

## Table 1

### Comparative gel filtration resins

| | No. of runs | Pool 1 | | | Pool 2 Yield | Total Recovery VIII:C |
|---|---|---|---|---|---|---|
| | | Yield | Sp.Act. | Purifi-cation | | |
| Pharmacia Cl 2B® | 6 | 27% | 7.6 | 13x | 61% | 88% |
| Pharmacia Cl 4B® | 7 | 54% | 12.8 | 21.4x | 37.5% | 91% |
| Pharmacia Cl 6B® | 3 | 37% | 6.8 | 11.3x | 55% | 82% |
| BioGel A-50M® (100-200 Mesh) | – | 61% | 11.2 | 17.2x | 51% | 99% |
| BioGel A-5M® (100-200 Mesh) | – | 67% | 15.7 | 24.1x | 41% | 103% |
| BioGel A-5M® (200-400 Mesh) | 5 | 66% | 14 | 21.8x | 34% | 99% |
| BioGel A-15M® (200-400 Mesh) | 6 | 51.2% | 12.6 | 21.2x | 32% | 83.1% |
| BioGel A-50M® (100-200 Mesh) | 6 | 44% | 10 | 17x | 51% | 96% |
| BioGel A-150M® | 5 | 22% | 6.1 | 11x | 74% | 96% |
| LKB Ultrogel A4® | 5 | 64% | 13 | 21x | 41% | 100% |
| CPG – 75 | 3 | 14% | .57 | – | 77% | 91% |
| CPG – 500 | 6 | 55% | 4.9 | 4.9x | 31% | 86% |
| CPG – 1000 | 5 | 34% | 15 | 26x | 60% | 93% |
| Fractogel TSK-65® | 6 | 30% | 5.5 | 5.4x | 53% | 83% |
| Fractogel TSK-75® | 7 | 30.4% | 14 | 10x | 52% | 82% |

## Example 3

A 150 ml sample of an AHF pool from a BioGel A5M column run as described above was diluted with column buffer to 700 ml. To the diluted AHF pool was added 0.9M L-Glycine, 0.8 M L-Lysine 0.002M $CaCl_2$ and 1.2g/ml sucrose, as described in U.S. Patent 4,543,210. After mixing to solubilize, the solution was placed in a 60° C water bath for 10.5 hours, 1/2 hour warm-up (wet heat pasteurization). After the 60° C incubation the pasteurized solution was diluted 1:1 with column buffer and prefiltered through an AP25 flat stock filter. The effluent was DF/UF against 8L of WFI at room temperature, followed by 2 - 3 volumes of column buffer. The DF-pasteurized AHF was then sterile filtered and freeze dried. The yield through the pasteurization DF/UF steps was 75%. The specific activity of the final container was found to be 43.1 units per $A_{280}$ unit. No HSA had been added in this particular run.

This Example demonstrates that an AHF pool from the present gel filtration column may be heat treated as an additional or alternative viral inactivation step.

Example 4

Increasing levels of sucrose were added to a sample of AHF pool from a BioGel® A5M column run. To determine the effect of protein concentration, both undiluted and 1:1 dilutions of the AHF pool were used. Other excipients included either 0.3 M L-glycine, 0.5 M L-lysine(1) or 0.9 M L-glycine or 0.5 M, 0.8 M L-lysine(2). From 0.2g/ml of sucrose to 1.2g/ml of sucrose (horizontal axis) were added and the samples were heated for 10 hours at 60° C in a water bath. Pre and Post Factor VIII assays were performed on the samples. In Figure 1, the AHF yield vs. the level of sucrose indicate that the level of sucrose can be reduced from 1.2g/ml to 0.6g/ml because the AHF recoveries are approximately the same from 0.6 to 1.2g/ml of sucrose.

With reference to the drawing, the legend "OLD ST" refers to an undiluted AHF pool containing excipients (1) prior to sucrose addition. The legend "OLD DIL" refers to a 1:1 AHF pool diluted with column buffer in excipients (1) prior to sucrose addition. "NEW DIL" similarly refers to a 1:1 diluted AHF pool in excipients (2) prior to sucrose addition. "NEW ST" refers to undiluted AHF pool in excipient (2).

Sucrose levels down to 0.6 g/ml showed not more than 30% loss of activity with either excipients (1) or (2).

Excipients (2) showed nearly 20% yield improvement over excipients (1) at 0.6 g sucrose/ml.

This Example shows that gel-filtered AHF can be wet heat treated with reduced sucrose levels, i.e., below 1.2, or 1.1 - 0.6 mg/ml. Reducing the sucrose levels will reduce processing (DF/UF). It can also enhance viral inactivation since sucrose protects virus particles as well as AHF. The lower sucrose level would provide less protection for the viral particle without loss of AHF activity. Identical results for the undiluted/diluted AHF samples were seen in both buffer systems.

Example 5

The AHF pool from a production column run was ultrafiltered (UF) using Amicon hollow fiber cartridges 10 x 9.29 dm² (10 sq. ft.). The AHF pool (16.2 Kg) was ultrafiltered in 1 hour to a weight of 4.8 Kg. The following table summarizes the pertinent data for the ultrafiltration step.

## Table II
### Ultrafiltration of Gel Filtered AHF

| Step | Weight (Kg) | $A_{280}$ | AHF (u/ml) | Specific Activity | Total AHF (units) | Yield (%) |
|------|------|------|------|------|------|------|
| AHF Pool (1) | 16.2 | 0.93 | 57.1 | 61.4 | 925,020 | --- |
| U.F. Pool (1) | 4.8 | 2.95 | 182.4 | 61.8 | 875,520 | 94.7 |

The AHF pool was U.F. very easily with no loss in purity and very little loss in yield (approximately 5%). The AHF potency was concentrated to greater than 180 units per ml. In separate experiments it has been possible to easily ultrafilter AHF Pool (1) to greater than 300 units of AHF per ml. At this high potency, a very low volume of reconstituted final container will enable the hemophiliac to receive a large quantity of AHF quickly. The final container potency will depend upon the extent of ultrafiltration. Expected range of final container potencies is between 50 to 300 units per ml of AHF.

Example 6

The ultrafiltered AHF Pool (1) from Example 5 was dilutd with column buffer and normal serum albumin was added such that the calculated final container potency would be approximately 100 u/ml. After sterile filtration (as in Example 1) and lyophilization, the final container AHF concentrate was assayed, and some of these results are tabulated in Table III.

## Table III

### Final Container Test Results on TNBP/Tween®AHF

| Test | Results |
|------|---------|
| AHF Potency | 104 $\mu$/ml |
| von Willebrand Factor | 95 $\mu$/ml |
| Specific Activity | 16.8 units/mg protein |
| TNBP | $\leq$ 0.8 ppm |
| Tween®80 | $\leq$ 0 ppm |
| Rabbit Pyrogen | pass |
| Sterility | pass |
| Safety | pass |
| Fibronectin | 0.39 mg/ml |
| Fibrinogen | > 0.6 mg/ml |
| IgG | > 0.015 mg/ml |

As can be seen in the table, an AHF concentrate can be prepared at 4 times the usual .25 u/ml dose and not affect the final container properties. There was no problem in sterile filtering this AHF pool. The rabbit pyrogen test was performed by injecting 100 units AHF per Kg of rabbit and the total temperature use in three rabbits was only 0.3° C. The calculated ratio of AHF to von Willebrand Factor of 1.1 implies an almost ideal plasma ratio of 1.0 in the final container. Non-detectible TNBP and Tween 80 were found in this final container AHF concentrate.

## Table IV

| | Lot 1 | Lot 2 | Lot 3 | Lot 4 | Lot 5 |
|---|---|---|---|---|---|
| Fill size, ml/vial | 10 | 10 | 40 | 20 | 20 |
| AHF, u/ml | 36 | 32 | 27.5 | 24.7 | 30.3 |
| Protein, mg/ml | 5.4 | 5.3 | 5.2 | 5.2 | 5.3 |
| Pool 1 specific activity | 55 | 47.2 | 47.2 | 44.3 | 46.8 |
| Specific activity | 6.7 | 6 | 5.3 | 4.8 | 5.7 |
| Ratio VIII RcoF/VIII:C | 1.4 | 1 | 1.5 | 1.7 | 1 |
| Glycine % | 0.37 | 0.38 | 0.37 | 0.01 | 0.03 |
| Units AMF/mg Fibrinogen | >60 | >53.3 | >45.8 | >41.2 | >50.4 |
| Fibronectin, mg/ml | 0.8 | 0.11 | 0.05 | 0.03 | 0.1 |
| Fibrinogen, mg/ml | <0.6 | <0.6 | <0.6 | <0.6 | <0.6 |
| IgA, mg/ml | <0.05 | <0.05 | <0.05 | <0.05 | <0.05 |
| IgG, mg/ml | <0.015 | <0.015 | <0.015 | <0.015 | <0.015 |

Table IV shows additional assay results from various lots. The ratio of AHF to vWF is shown as Ratio VIII RcoF/VIII:C.

Thus there has been described a process for the preparation of AHF comprising a sequence of precipitation, solubilization, gel filtration and viral inactivation steps. Notwithstanding that reference has been made to specific preferred embodiments, it will understood that the present invention is not be construed as limited to such, but rather to the lawful scope of the appended claims.

## Claims

1. A process for the production of a concentrate of antihemophilic factor (AHF) from cryoprecipitate without substantial loss of therapeutic or immunological activity comprising the sequential steps of:
   (a) dissolving said cryoprecipitate;
   (b) removing non-AHF proteins by precipitation with polyethylene glycol (PEG) without a chill step;
   (c) heat treating said AHF for viral inactivation in the presence of sucrose, the amount of sucrose being 1.2 to 0.6 g/ml of pooled concentrate; and then
   (d) passing said AHF through a gel filtration column containing a size exclusion resin from 300 to 15,000 daltons to concentrate said AHF to at least 35 units of Factor VIII activity per ml of pooled concentrate.

2. The process of claim 1 further comprising the step of precipitating AHF with a mixture of glycine and NaCl prior to said gel filtration.

3. The process of claim 1 wherein PEG is added at step (b) in an amount of 2% - 5%.

4. The process of claim 1 further comprising the step of ultrafiltering said concentrate to obtain at least 100 units of Factor VIII activity per ml of concentrate.

5. A process of claim 1 for the production of antihemophilic factor concentrate (AHF) from cryoprecipitate without substantial loss of therapeutic or immunological activity comprising the sequential steps of:
   (a) preparing a cryoprecipitate;

(b) removing non-AHF proteins by precipitation with polyethylene glycol (PEG) without a chill step to form a solubilized AHF pool;

(c) heating said AHF obtained from the column for viral inactivation in the presence of sucrose, the amount of sucrose being 1.2 to 0.6 g/ml of pooled concentrate; and then

(d) passing said AHF pool through a gel filtration column containing a size exclusion resin from 300 to 15,000 daltons to concentrate said AHF to at least 35 units of Factor VIII activity per ml of pooled concentrate.

6. The process of claim 5 further comprising the step of, between steps (b) and (c), concentrating said pool by ultrafiltration.


**Patentansprüche**

1. Verfahren zur Herstellung eines Konzentrats von antihämophilem Faktor (AHF) aus Kryopräzipitat ohne wesentlichen Verlust an therapeutischer oder immunologischer Aktivität, wobei die folgenden aufeinanderfolgenden Stufen umfaßt sind:

(a) Auflösen des genannten Kryopräzipitats;

(b) Entfernen von nicht-AHF-Proteinen durch Ausfällung mit Polyethylenglykol (PEG) ohne eine Kühlstufe;

(c) Wärmebehandeln des genannten AHF zur viralen Inaktivierung in der Gegenwart von Sucrose, wobei die Menge an Sucrose 1,2 bis 0,6 g/ml gepooltes Konzentrat beträgt; und dann

(d) Laufenlassen des genannten AHF durch eine Gelfiltrationssäule, enthaltend ein Größenausschlußharz von 300 bis 15.000 Daltons, um den genannten AHF auf mindestens 35 Einheiten an Faktor VIII-Aktivität pro ml gepooltes Konzentrat aufzukonzentrieren.

2. Verfahren gemäß Anspruch 1, wobei ferner die Stufe einer Ausfällung von AHF mit einer Mischung aus Glycin und NaCl vor der genannten Gelfiltration umfaßt ist.

3. Verfahren gemäß Anspruch 1, worin PEG in Stufe (b) in einer Menge von 2 % bis 5 % zugefügt wird.

4. Verfahren gemäß Anspruch 1, wobei ferner die Stufe einer Ultrafiltration des genannten Konzentrats umfaßt ist, um mindestens 100 Einheiten an Faktor VIII-Aktivität pro ml Konzentrat zu erhalten.

5. Verfahren gemäß Anspruch 1 zur Herstellung eines Antihämophilfaktor-Konzentrats (AHF) aus Kryopräzipitat ohne wesentlichen Verlust an therapeutischer oder immunologischer Aktivität, wobei die folgenden aufeinanderfolgenden Stufen umfaßt sind:

(a) Herstellen eines Kryopräzipitats;

(b) Entfernen von nicht-AHF-Proteinen durch Ausfällung mit Polyethylenglykol (PEG) ohne eine Kühlstufe, um einen solubilisierten AHF-Pool zu bilden;

(c) Erwärmen des aus der Säule zur viralen Inaktivierung erhaltenen AHF in der Gegenwart von Sucrose, wobei die Menge an Sucrose 1,2 bis 0,6 g/ml gepooltes Konzentrat beträgt; und dann

(d) Laufenlassen des genannten AHF-Pools durch eine Gelfiltrationssäule, enthaltend ein Größenausschlußharz von 300 bis 15.000 Daltons, um den genannten AHF auf mindestens 35 Einheiten an Faktor VIII-Aktivität pro ml gepooltes Konzentrat aufzukonzentrieren.

6. Verfahren gemäß Anspruch 5, wobei ferner zwischen den Stufen (b) und (c) die Stufe einer Aufkonzentrierung des genannten Pools durch Ultrafiltration umfaßt ist.


**Revendications**

1. Procédé de production d'un concentré de facteur antihémophilique (AHF) à partir du cryoprécipité sans perte notable d'activité thérapeutique ou immunologique, comprenant les étapes successives :

(a) de dissolution dudit cryoprécipité ;

(b) d'élimination des protéines ne consistant pas en AHF par précipitation avec le polyéthylèneglycol (PEG) sans étape de refroidissement,

(c) de traitement thermique dudit AHF pour l'inactivation virale en présence de saccharose, la quantité de saccharose étant comprise dans l'intervalle de 1,2 à 0,6 g/ml de concentré total ; puis

(d) de passage dudit AHF à travers une colonne de filtration sur gel contenant une résine d'exclusion dimensionnelle de 300 à 15 000 daltons pour concentrer ledit AHF à au moins 35 unités d'activité de Facteur VIII par ml de concentré total.

2. Procédé suivant la revendication 1, comprenant en outre l'étape de précipitation du AHF avec un mélange de glycine et de NaCl avant la filtration sur gel.

3. Procédé suivant la revendication 1, dans lequel le PEG est ajouté à l'étape (b) en une quantité de 2 % à 5 %.

4. Procédé suivant la revendication 1, comprenant en outre l'étape d'ultrafiltration du concentré pour obtenir au moins 100 unités d'activité de Facteur VIII par ml de concentré.

5. Procédé suivant la revendication 1 pour la production d'un concentré de facteur antihémophilique (AHF) à partir du cryoprécipité sans perte notable d'activité thérapeutique ou immunologique, comprenant les étapes successives :
   (a) de préparation d'un cryoprécipité ;
   (b) d'élimination des protéines ne consistant pas en AHF par précipitation avec le polyéthylèneglycol (PEG) sans étape de refroidissement pour former un volume total de AHF solubilisé ;
   (c) de chauffage dudit AHF obtenu à partir de la colonne pour l'inactivation virale en présence de saccharose, la quantité de saccharose étant comprise dans l'intervalle de 1,2 à 0,6 g/ml de concentré total ; puis
   (d) de passage dudit volume total de AHF à travers une colonne de filtration sur gel contenant une résine d'exclusion dimensionnelle de 300 à 15 000 daltons pour concentrer ledit AHF à au moins 35 unités d'activité de Facteur VIII par ml de concentré total.

6. Procédé suivant la revendication 5, comprenant en outre l'étape, entre les étapes (b) et (c), de concentration du volume total par ultrafiltration.

Wet Heat Studies

FIG.1

□ Old ST          + Old DIL          o New DIL          △ New ST

EP 0 399 321 B1